# FASCICULE DE BREVET EUROPEEN

(11) **EP 1 575 670 B1**
(45) Date de publication et mention de la délivrance du brevet: **29.09.2010**
(21) Numéro de dépôt: 03813932.5
(22) Date de dépôt: 22.12.2003
(51) Int. Cl.: A61P 1/02

(54) **COMPOSITION HUILEUSE A BASE DE LIPIDES PEROXYDES UTILISABLE DANS LE TRAITEMENT DE LA XEROSTOMIE**
ÖLHALTIGE ZUSAMMENSETZUNG AUF BASIS VON PEROXIDIERTEN LIPIDEN FÜR XEROSTOMIE
OIL COMPOSITION BASED ON PEROXIDISED LIPIDS, WHICH CAN BE USED IN THE TREATMENT OF XEROSTOMIA

(30) Priorité: 23.12.2002 FR 0216517
(43) Date de publication de la demande: 21.09.2005
(73) Titulaire: Laboratoires Carilene, 78360 Montesson (FR)
(72) Inventeur: DESJONQUERES, Stéphane, F-78600 Maisons-Laffitte (FR)
(74) Mandataire: Giraud, Françoise
(86) Numéro de dépôt international: PCT/FR2003/003861
(87) Numéro de publication internationale: WO 2004/058138

(56) Documents cités:
- EP-A- 0 225 832
- EP-A- 1 077 061
- FR-A- 2 705 568

## Description

La présente invention concerne une nouvelle composition à base d'huiles peroxydées utilisable notamment dans le traitement de la xérostomie.

La xérostomie est une pathologie bien connue qui se manifeste par une sensation de sécheresse au niveau de la bouche.

Cette pathologie résulte généralement d'une paresse ou d'un dégât définitif des glandes salivaires qui provoque ce phénomène.

Ce syndrome gênant peut devenir particulièrement invalidant. Une sensation de brûlure et une douleur diffuses dans la cavité buccale gênent considérablement le patient pour s'alimenter ou pour boire.

Les manifestations de xérostomie s'observent le plus souvent après un traitement à long terme par des neuroleptiques, un traitement par irradiation ou chimiothérapie ou dans le cas d'imuno-déficience.

Actuellement, il n'existe aucun traitement satisfaisant mais seulement certains produits agissant sur les symptômes en augmentant la sécrétion salivaire, en se substituant à la salive ou en stimulant les glandes salivaires. Le principal traitement actuellement utilisé consiste à utiliser des salives artificielles ou substituts salivaires qui sont pulvérisés dans la bouche.

D'une façon générale, ces produits sont avalés sans beaucoup de rémanence thérapeutique.

Ainsi, de nombreux substituts de salive sont commercialisés.

La formulation des substituts de la salive tend habituellement à s'approcher de la composition de la salive naturelle.

On connaît également des produits destinés à stimuler les glandes salivaires.

Il s'agit généralement de produits de type gommes à mâcher ou de pastilles.

On connaît différents lipides peroxydés, notamment obtenus par peroxydation d'huiles végétales naturelles. On citera, en particulier, les brevets suivants BSM N°2 330 M, EP-A-293 535, FR-A-2 591 112, EP-A-225 831, EP-A-225 832, EP-A-225 833, EP-A-226 506, FR-A-2 461 744, FR-A-2 539 142 et EP-A-117 962 qui concernent soit la préparation de tels lipides peroxydés soit leurs applications dans différents domaines, en particulier dans le traitement de certaines affections dans le domaine de la rhumatologie ou de la traumatologie, ou encore en tant que produit cicatrisant.

On a également décrit dans la demande de brevet européen EP 1077061 l'utilisation de lipides peroxydés dans le traitement ou la prévention de plaies et d'inflammations des muqueuses de la cavité buccale

Dans le cadre de ses recherches sur de nouveaux moyens de traitement du syndrome de bouche sèche, l'inventeur de la présente invention a maintenant découvert que les lipides peroxydés, à condition d'être formulés sous forme d'une composition compatible avec une application sous forme de spray, pouvaient être utilisés de façon particulièrement efficace dans le traitement de la xérostomie, cette formulation sous forme de spray permettant de tapisser l'ensemble de la cavité buccale et de la langue par une simple vaporisation de ladite composition dans la bouche.

Il est ainsi maintenant apparu, qu'à condition de formuler les lipides peroxydés sous forme d'une composition présentant une viscosité bien déterminée, liée à une présence de silice dans des proportions bien déterminées dans la composition, il était possible de tapisser l'ensemble des muqueuses de la bouche de façon particulièrement simple et efficace au moyen d'une pompe doseuse, ce qui conduisait à la formation d'un film lipidique tapissant l'ensemble des muqueuses et provoquant une véritable action de lubrification des muqueuses de la bouche.

Ce type de composition et de mode d'action s'avère particulièrement intéressant, à une époque où l'on recherche particulièrement des modes de traitement aussi peu agressifs que possible pour l'organisme. Ils permettent, en outre, de satisfaire à la réglementation de la Communauté Européenne concernant les dispositifs médicaux.

Par ailleurs, on notera que, parmi les documents cités précédemment décrivant des compositions à base de lipides peroxydés, il en existe un certain nombre qui citent des compositions contenant de la silice colloïdale. Toutefois, dans tous ces documents citant des mélanges de lipides peroxydés et de silice colloïdale, on cherche à faire des compositions visqueuses ayant la consistance d'un gel huileux applicable sur la peau et les muqueuses.

Ces gels contiennent toujours des concentrations en silice colloïdale supérieures à 4% en poids et généralement supérieures à 6% en poids. De telles compositions ne permettraient pas de remplir la fonction visée selon la présente invention où l'on cherche à tapisser totalement les muqueuses de la cavité buccale en vaporisant la composition sous forme de spray.

Ainsi donc, l'invention concerne, à titre de produit nouveau, des compositions à base de lipides peroxydés et de silice colloïdale dans des proportions bien déterminées ainsi que des utilisations de ces compositions.

Ainsi, l'invention propose une composition à base de lipides peroxydés qui peut être aisément diffusée sous forme de spray dans la cavité buccale et n'est ni un substitut ni un stimulant de la salive. Il s'agit plutôt d'un agent lubrifiant buccal doté d'une propriété d'adhérence à la muqueuse buccale, formant un film protecteur sur l'ensemble des muqueuses de la bouche.

Le film lipidique qui se forme évite la perte d'humidité des tissus buccaux, réduisant ainsi la tendance à la sécheresse de la muqueuse buccale chez des patients ayant une diminution de la fonction salivaire.

Ces propriétés confèrent des avantages notables à l'utilisation de la composition sous forme de spray à base de lipides peroxydés chez des patients souffrants de sécheresse buccale.

Plus précisément, les compositions de la présente invention contiennent, comme les compositions préférées décrites dans le brevet EP 1077061, de la silice en tant qu'agent épaississant.

Toutefois, la viscosité des compositions de la présente invention est moindre, de façon à permettre son utilisation sous forme de spray buccal et non sous forme de gel buccal.

Ainsi, les gels décrits dans la demande EP 1077061 sont particulièrement indiqués dans le cas où l'on cherche une utilisation sous forme de gel topique pour le soulagement symptomatique des ulcérations buccales, des gingivites et des douleurs consécutives au port d'appareils dentaires, alors que les nouvelles compositions selon l'invention, présentent, du fait de leur faible viscosité, la possibilité d'être utilisées pour tapisser l'ensemble de la cavité buccale, ce qui conduit à une nouvelle indication des lipides peroxydés dans le traitement de la xérostomie.

Un avantage tout particulier des compositions selon l'invention est qu'elles n'induisent aucun effet pharmacologique, métabolique ou immunologique puisqu'elles ne contiennent pas de composés pharmacologiques et sont constituées essentiellement de triesters de glycérol oxydés et de dioxyde de silicium, les autres constituants étant pour l'essentiel, des arômes alimentaires.

Les observations faites sur des patients souffrant de xérostomie indiquent que ces compositions sont particulièrement efficaces pour soulager tous les symptômes associés à la sécheresse buccale.

D'autres avantages et caractéristiques de la présente invention apparaîtront clairement au vu de la description et des exemples qui suivent.

Selon l'une de ses caractéristiques essentielles, l'invention concerne une composition pharmaceutique huileuse à base de lipides peroxydés et de silice contenant, à titre de constituants essentiels, des lipides peroxydés présentant un taux de peroxydation compris entre 5 et 600 milli-équivalents par kilo et de la silice dispersée au sein desdits lipides peroxydés, à une teneur supérieure ou égale à 0,5% en poids et inférieure à 4% en poids par rapport au poids total de ladite composition.

Par "constituants essentiels" au sens de l'invention, il faut comprendre que les lipides peroxydés et la silice dans des proportions bien déterminées sont les constituants de base de la composition qui est une composition huileuse de viscosité bien déterminée. Toutefois, la composition de l'invention pourra en outre contenir d'autres constituants compatibles avec une utilisation buccale tels que :
- un agent anti-bactérien compatible avec une utilisation buccale, par exemple la chlorhexidine,
- un agent antimycosique qui pourra être tout agent anti-fongique à usage local compatible avec une utilisation buccale, par exemple un dérivé imidazolé ou un agent antibiotique antifongique de contact,
- un agent améliorant la tolérance de la composition dans la bouche, par exemple tout constituant connu comme facteur de tolérance ou comme agent adoucissant,
- un agent stabilisateur de la formule, par exemple un agent tampon, un agent épaississant ou fluidifiant ou un correcteur de pH,
- un agent protecteur de la formule, tel qu'un agent à effet antibactérien ou un conservateur, par exemple un ester de l'acide p-hydroxybenzoique, par exemple le parahydroxybenzoate de méthyle, le parahydroxybenzoate de propyle ou l'isothiazolinone,
- une composition parfumante, par exemple un arôme de type alimentaire, une huile essentielle, un extrait de plante ou de fruit.

Selon une autre de ses caractéristiques essentielles, l'invention concerne l'utilisation des lipides peroxydés pour la fabrication d'une composition pharmaceutique destinée au traitement de la xérostomie.

Les lipides peroxydés utilisés pour la préparation des compositions de l'invention résultent de la peroxydation de corps gras insaturés. Le degré de peroxydation est mesuré selon la norme ISO 3960.

Pour la préparation des compositions de la présente invention, on choisira des lipides peroxydés présentant un taux de peroxydation compris entre 5 et 600 milli-équivalents par kilo, de préférence entre 30 et 500 milli-équivalents par kilo.

Ce taux de peroxydation sera de façon encore plus avantageuse compris entre 50 et 300 milli-équivalents par kilo, de préférence encore entre 50 et 150 milli-équivalents par kilo.

Les lipides peroxydés préférés utilisés selon l'invention résultent de la peroxidation de lipides ou corps gras d'origine naturelle, de préférence d'origine végétale, de préférence encore de lipides issus d'une huile végétale naturelle.

A titre d'exemples d'huile naturelle choisie selon l'invention, on citera, l'huile d'amande douce, l'huile de noisette, l'huile d'arachide, l'huile de maïs, l'huile de pépin de raisin, l'huile de sésame et l'huile de carthame. On pourra également utiliser un mélange de ces huiles.

Selon une variante particulièrement préférée de l'invention, on choisira l'huile de maïs peroxydée et tout particulièrement une huile de maïs présentant un taux de peroxydation compris entre 5 et 600 milli-équivalents par kilo.

Les lipides peroxydés utilisés selon l'invention sont d'une façon avantageuse constitués, à titre de constituants majoritaires, représentant généralement au moins 80 % de la masse de triglycérides répondant à la formule 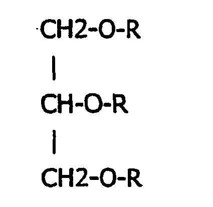 dans laquelle les radicaux R sont majoritairement représentés par des acides insaturés en C18 partiellement peroxydés (en fonction du taux de peroxydation dudit lipide).

Pour des raisons analytiques qui rendent difficile la détermination du degré de peroxydation dans les compositions, en présence de silice, on caractérisera souvent les compositions de l'invention, non pas à partir du taux de peroxydation du lipide peroxydé entrant dans ladite composition mais par la teneur en glycérides oxydés de la composition, teneur qui, bien entendu, est directement liée au taux de peroxydation du lipide peroxydé utilisé pour sa préparation.

Ainsi, cette teneur en glycérides oxydés des compositions de l'invention est généralement comprise entre 7 et 15% en poids.

Comme exposé précédemment, il est essentiel que les compositions de l'invention présentent une viscosité bien spécifique permettant d'obtenir, lors de leur vaporisation sous forme de spray dans la cavité buccale, au moyen d'une pompe doseuse, un tapissage aussi complet que possible des muqueuses buccales et de la langue.

Cette viscosité spécifique est conférée à la composition par la présence de silice dans des proportions bien déterminées.

Les compositions de l'invention contiennent ainsi moins de 4% et au moins 0,5% en poids de silice dispersée dans le lipide peroxydé.

Les concentrations préférées en silice sont avantageusement comprises entre 0,5 et 3,5% en poids, de préférence entre 0,5 et 2% en poids.

Il s'est avéré particulièrement avantageux d'utiliser des qualités de silice dites silice colloïdale, c'est à dire des silices dont le diamètre des particules correspond à environ 1/30 de la longueur d'onde de la lumière visible. Il s'agit de particules de silice amorphe présentant des diamètres d'environ 7 à 40 nm.

De telles particules de silice sont bien connues pour pouvoir être utilisées pour augmenter la viscosité des milieux dans lesquelles elles sont introduites.

Un exemple de silice colloïdale utilisable selon la présente invention est le produit commercialisé par la société Degussa sous la marque AEROSIL 300^{®}.

On notera toutefois que, pour une même concentration en silice, la viscosité de la composition dépend du procédé utilisé pour la dispersion de la silice dans l'huile peroxydée. D'autre part, il est apparu que la viscosité de la composition était sujette à des variations au cours du temps.

Dans ces conditions, la rhéologie des compositions utiles selon l'invention sera caractérisée sans ambiguïté par la viscosité de la composition au moment de sa fabrication.

Par ailleurs, il est important de noter que la viscosité de la composition, même si elle est liée à la concentration en silice dans la composition dépend également grandement du procédé utilisé pour disperser la silice au sein de la composition, en particulier de l'agitation appliquée et des conditions de température lors de cette dispersion.

Dans ces conditions, une autre façon de caractériser la rhéologie des compositions en recourant à une caractéristique plus stable au cours du temps est de caractériser la composition par sa densité.

Ainsi, les compositions de l'invention auront avantageusement, au moment de leur préparation, une viscosité cinématique mesurée conformément à la pharmacopée européenne par une méthode au tube capillaire, à 20°C, comprise entre 26,6 et 44,4 mm²/s.

Ces compositions auront également, de façon préférée, une densité de 0,95 ± 10%.

Comme exposé précédemment, la composition galénique de la composition pharmaceutique de la présente invention est particulièrement importante puisque la rhéologie, caractérisée par la mesure de la viscosité initiale et de la densité de la composition, s'avère être une des caractéristiques essentielles de la composition de l'invention permettant d'obtenir l'effet recherché.

En effet, la silice confère un certain épaississement à l'huile peroxydée qui, du fait de sa teneur bien spécifique en silice se présente sous une forme qui demeure parfaitement fluide, conférant à la composition de bonnes propriétés de répartition dans l'ensemble de la cavité buccale, ce qui permet à la composition de tapisser l'ensemble de la muqueuse de la bouche et de la langue et de rester en place après vaporisation de la composition dans la cavité buccale.

C'est cette viscosité spécifique qui permet l'utilisation des lipides peroxydés sous forme de spray.

Le produit doit en effet être suffisamment fluide pour être vaporisé à travers la buse et la pompe de vaporisation mais suffisamment visqueux pour tapisser ensuite tous les tissus de la cavité buccale.

Le produit de l'invention est avantageusement présenté en flacon muni d'un dispositif de pompe permettant de vaporiser la composition dans la bouche. La qualité de la pompe et le diamètre de la buse sont choisis de façon à assurer une bonne dispersion sous forme d'un jet éclaté dans la bouche, conduisant à un fin brouillard qui vient ensuite tapisser l'ensemble des muqueuses de la bouche.

On choisira de préférence une pompe permettant de disperser, à chaque pression exercée, des quantités de l'ordre de 100 µL.

### EXEMPLES :

Sauf indications contraires, les proportions données dans les exemples qui suivent sont exprimées en pourcentage en poids.

### EXEMPLE 1

### Spray buccal

### a. Composition

■ Huile de maïs peroxydée présentant un taux de peroxidation compris entre 50 et 150 milli-équivalents par kilo 94,4%
■ Dioxyde de silicium (AEROSIL 300^{®} - degussa) : 1,5%
■ Arôme alimentaire, menthe : 1,0%
■ Arôme alimentaire, orange-pamplemousse : 3,0%
■ Aspartame : 0,1%

Le produit se présente sous forme d'un gel fluide de couleur jaune-jaune d'or présentant une odeur mentholée et d'agrumes et un goût de menthe-pamplemousse.

Ce produit présente une densité de l'ordre de 0,925 et une viscosité de 35,5 mm²/s mesuré à l'aide d'un tube type capillaire, à 20°C.

### EXEMPLE 2

### Test d'efficacité sur la xérostomie.

### a. Objectif du test

Le test consiste à évaluer l'effet des triglycérides oxydés chez des utilisateurs présentant un symptôme de sécheresse buccale.

### b. Protocole et conditions du test

Le produit testé est pulvérisé dans la bouche des utilisateurs sous forme d'un fin brouillard à l'aide d'un dispositif muni d'une pompe et d'une buse, dispersant 100 µL à chaque pression et l'application est renouvelée aussi souvent que nécessaire. Une ou deux pulvérisations sont réalisées après chaque repas et aussi souvent que nécessaire et le patient se rince bien la bouche avant chaque application.

Le test a été mené sur une période de 15 jours.

L'intensité de la gêne provoquée par le syndrome de bouche sèche a été évaluée avant (temps J₀) et après traitement (J + 15) à l'aide de droites analogiques de 10 cm sur lesquelles chaque expérimentateur et chaque utilisateur notent l'intensité de la gêne, de légère à très intense.

Le test a été réalisé par plusieurs expérimentateurs en milieu libéral et tout particulièrement en psychiatrie et en cancérologie et a porté sur 49 utilisateurs dont 34 suivaient des traitements psychiatriques ou par psychotropes et 11 des traitements anti-cancéreux par irradiation ou chimiothérapie.

Au temps J₀, il a été établi que 63,26 % des utilisateurs souffraient de symptômes importants à très importants.

Par ailleurs, 75,51 % des utilisateurs étaient traités antérieurement par un produit commercial substitut de la salive alors que 24,49 % des utilisateurs ne recevaient aucun traitement avant le début du test.

### c. Résultats obtenus en utilisant la composition de l'exemple 1

### c1. Evaluation faite par les utilisateurs

L'importance de la gêne évaluée par l'utilisateur à J₀ sur une échelle analogique de 10 centimètres correspond à une valeur moyenne de 6,34.

L'importance de la gêne évaluée par l'utilisateur à J + 15 sur une échelle analogique de 10 centimètres correspond à une valeur moyenne de 3,82.

La comparaison des valeurs moyennes obtenues par les utilisateurs à J + 15 montre que 87,75 % des utilisateurs ont pu constater une amélioration significative de leurs syndromes de bouche sèche.

L'évaluation de la diminution de l'importance de la gêne montre une diminution moyenne de 2,98 unités sur l'échelle analogique. L'intensité des symptômes a donc diminué de 47 % en moyenne.

### c2. Evaluation faite par les expérimentateurs

Les observations cliniques réalisées par les expérimentateurs confirment les effets bénéfiques rapportés par les utilisateurs.

En effet, les expérimentateurs ont attribué une valeur moyenne de 6,06 à la gêne évaluée par l'expérimentateur à J₀, sur une échelle analogique de 10 cm et une valeur moyenne de 3,46 à l'importance de la gêne évaluée par l'expérimentateur à J+15 sur une échelle analogique de 10 cm et les expérimentateurs ont pu constater une amélioration significative du syndrome de bouche sèche dans 91, 83% et une diminution moyenne de l'intensité des symptômes de 48, 84 %.

Par ailleurs, on note une durée de soulagement du syndrome de bouche sèche de plus de 3 heures pour 83,67 % des utilisateurs alors que l'administration par voie orale ou la pulvérisation d'une salive artificielle ne procure à l'utilisateur qu'un bénéfice fugace.

## Revendications

1. Composition pharmaceutique huileuse à base de lipides peroxydés et de silice **caractérisée en ce qu'**elle contient, à titre de constituants essentiels, des lipides peroxydés présentant un taux de peroxydation compris entre 5 et 600 milli-équivalents par kilo et de la silice dispersée au sein desdits lipides peroxydés, à une concentration en poids, supérieure ou égale à 0,5% et inférieure à 4%.

2. Composition pharmaceutique selon la revendication 1, **caractérisée en ce qu'**elle contient de 0,5 à 3,5% en poids de silice.

3. Composition pharmaceutique selon la revendication 1 ou 2, **caractérisée en ce qu'**elle contient de 0,5 à 2% en poids de silice.

4. Composition pharmaceutique selon l'une des revendications 1 à 3, **caractérisée en ce qu'**elle est sous une forme compatible avec une utilisation sous forme de spray.

5. Composition pharmaceutique selon l'une des revendications 1 à 4, **caractérisée en ce que** la silice est sous forme de silice colloïdale.

6. Composition pharmaceutique selon l'une des revendications 1 à 5, **caractérisée en ce que** sa viscosité cinématique au moment de la fabrication de ladite composition, mesurée à 20°C au moyen d'un viscosimètre de type capillaire, est comprise entre 26,6 et 44,4 mm²/s.

7. Composition selon l'une des revendications 1 à 6, **caractérisée en ce que** sa densité est de 0,95 ±10%.

8. Composition selon l'une des revendications 1 à 7, **caractérisée en ce que** lesdits lipides peroxydés présentent un taux de peroxydation compris entre 5 et 600, de préférence entre 30 et 500, de préférence encore entre 50 et 300, de préférence encore entre 50 et 150 milli-équivalents par kilo.

9. Utilisation selon l'une des revendications 1 à 8, **caractérisée en ce que** lesdits lipides peroxydés comprennent, à titre de constituants majoritaires, des triglycérides partiellement oxydés répondant à la formule générale : 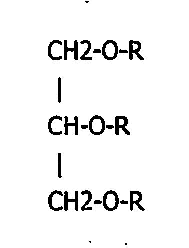 dans laquelle les radicaux R sont des acides insaturés en C18 partiellement peroxydés.

10. Composition pharmaceutique selon l'une des revendications 1 à 9, **caractérisée en ce que** lesdits lipides peroxydés sont obtenus par peroxydation de lipides ou de corps gras d'origine naturelle, de préférence de lipides issus d'une huile végétale naturelle.

11. Composition pharmaceutique selon la revendication 10, **caractérisée en ce que** l'huile naturelle est choisie dans le groupe constitué de l'huile d'amande douce, de l'huile de noisette, de l'huile d'arachide, de l'huile de maïs, de l'huile de pépin de raisin, de l'huile de sésame et de l'huile de carthame et de leurs mélanges.

12. Utilisation de lipides peroxydés tels que définis dans l'une des revendications 1 ou 8 à 11 pour la fabrication d'une composition pharmaceutique destinée au traitement de la xérostomie **caractérisée en ce que** ladite composition contient au moins 0,5% en poids et moins de 4% en poids de silice colloïdale, de préférence entre 0,5 et 3,5% en poids, de préférence encore entre 0,5 et 2% en poids.

## Claims

1. An oily pharmaceutical composition based on peroxidised lipids and on silica, **characterised in that** it contains, as essential constituents, peroxidised lipids which have a degree of peroxidation of between 5 and 600 milli-equivalents per kilogram, and silica which is dispersed within said peroxidised lipids at a concentration by weight of greater than or equal to 0.5% and less than 4%.

2. The pharmaceutical composition according to claim 1, **characterised in that** it contains 0.5 to 3.5% by weight of silica.

3. The pharmaceutical composition according to claim 1 or claim 2, **characterised in that** it contains 0.5 to 2% by weight of silica.

4. The pharmaceutical composition according to one of claims 1 to 3, **characterised in that** it is in a form which is compatible with a use as a spray.

5. The pharmaceutical composition according to one of claims 1 to 4, **characterised in that** the silica is in a form of colloidal silica.

6. The pharmaceutical composition according to one of claims 1 to 5, **characterised in that** its cinematic viscosity upon preparing said composition, measured at 20°C by means of a capillary viscometer, is between 26.6 and 44.4 mm²/s.

7. The composition according to one of claims 1 to 6, **characterised in that** its density is 0.95 ± 10%.

8. The composition according to one of claims 1 to 7, **characterised in that** said peroxidised lipids have a degree of peroxidation of between 5 and 600, preferably between 30 and 500, more preferably between 50 and 300, more preferably between 50 and 150 milli-equivalents per kilogram.

9. The composition according to one of claims 1 to 8, **characterised in that** said peroxidised lipids comprise, as main constituents, partially oxidised triglycerides of general formula: 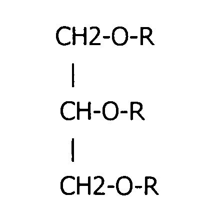 in which the radicals R are partially peroxidised C18 unsaturated acids.

10. The pharmaceutical composition according to one of claims 1 to 9, **characterised in that** said peroxidised lipids are obtained by peroxidation of lipids or of fats of natural origin, preferably of lipids originating from a natural plant oil.

11. The pharmaceutical composition according to claim 10, **characterised in that** the natural oil is selected from the group consisting of sweet almond oil, hazelnut oil, groundnut oil, maize oil, grapeseed oil, sesame oil, and safflower oil, and their mixtures.

12. Use of peroxidised lipids as defined in one of claims 1 or 8 to 11 for the preparation of a pharmaceutical composition intended for the treatment of xerostomia, **characterised in that** said composition contains at least 0.5% by weight and less than 4% by weight of colloidal silica, preferably between 0.5 and 3.5% by weight, more preferably between 0.5 and 2% by weight.

## Patentansprüche

1. Ölige pharmazeutische Zusammensetzung auf Basis von peroxidierten Lipiden und Siliziumoxid, **dadurch gekennzeichnet, daß** sie als wesentliche Bestandteile peroxidierte Lipide, die einen Peroxidationsgrad zwischen 5 und 600 Milliäquivalenten pro Kilo aufweisen, und in diesen peroxidierten Lipiden dispergiertes Siliziumoxid mit einer Gewichtskonzentration größer oder gleich 0,5 % und kleiner als 4 % enthält.

2. Pharmazeutische Zusammensetzung nach Anspruch 1, **dadurch gekennzeichnet, daß** sie 0,5 bis 3,5 Gew.-% Siliziumoxid enthält.

3. Pharmazeutische Zusammensetzung nach Anspruch 1 oder 2, **dadurch gekennzeichnet, daß** sie 0,5 bis 2 Gew.-% Siliziumoxid enthält.

4. Pharmazeutische Zusammensetzung nach einem der Ansprüche 1 bis 3, **dadurch gekennzeichnet, daß** sie in einer Form vorliegt, die mit einer Verwendung in Sprayform verträglich ist.

5. Pharmazeutische Zusammensetzung nach einem der Ansprüche 1 bis 4, **dadurch gekennzeichnet, daß** das Siliziumoxid in Form von kolloidalem Siliziumoxid vorliegt.

6. Pharmazeutische Zusammensetzung nach einem der Ansprüche 1 bis 5, **dadurch gekennzeichnet, daß** ihre kinematische Viskosität zum Zeitpunkt der Herstellung der Zusammensetzung, gemessen bei 20 °C mittels eines Viskosimeters vom Kapillartyp, zwischen 26,6 und 44,4 mm²/s liegt.

7. Zusammensetzung nach einem der Ansprüche 1 bis 6, **dadurch gekennzeichnet, daß** ihre Dichte 0,95 ± 10 % ist.

8. Zusammensetzung nach einem der Ansprüche 1 bis 7, **dadurch gekennzeichnet, daß** die peroxidierten Lipide einen Peroxidationsgrad zwischen 5 und 600, vorzugsweise zwischen 30 und 500, weiter bevorzugt zwischen 50 und 300, weiter bevorzugt zwischen 50 und 150 Milliäquivalenten pro Kilo aufweisen.

9. Zusammensetzung nach einem der Ansprüche 1 bis 8, **dadurch gekennzeichnet, daß** die peroxidierten Lipide als Hauptbestandteile partiell oxidierte Triglyceride umfassen, die der allgemeinen Formel entsprechen: 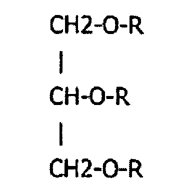 in welcher die Reste R ungesättigte, teilweise peroxidierte C18-Säuren sind.

10. Pharmazeutische Zusammensetzung nach einem der Ansprüche 1 bis 9, **dadurch gekennzeichnet, daß** die peroxidierten Lipide erhalten sind durch Peroxidation von Lipiden oder Fettkörpern natürlichen Ursprungs, vorzugsweise Lipiden aus einem Pflanzenöl.

11. Pharmazeutische Zusammensetzung nach Anspruch 10, **dadurch gekennzeichnet, daß** das natürliche Öl gewählt ist aus der Gruppe, die besteht aus Mandelöl, Nußöl, Erdnußöl, Maisöl, Traubenkernöl, Sesamöl und Safloröl und deren Gemischen.

12. Verwendung von peroxidierten Lipiden wie definiert in einem der Ansprüche 1 oder 8 bis 11 für die Herstellung einer pharmazeutischen Zusammensetzung, die vorgesehen ist zur Behandlung der Xerostomie, **dadurch gekennzeichnet, daß** die Zusammensetzung wenigstens 0,5 Gew.-% und weniger als 4 Gew.-%, vorzugsweise zwischen 0,5 und 3,5 Gew.-%, weiter bevorzugt zwischen 0,5 und 2 Gew.-% kolloidales Siliziumoxid enthält.
